(19) Europäisches Patentamt
European Patent Office
Office européen des brevets

(11) Veröffentlichungsnummer: **0 358 607**
**A2**

# (12) EUROPÄISCHE PATENTANMELDUNG

(21) Anmeldenummer: **89810648.9**

(22) Anmeldetag: **01.09.89**

(51) Int. Cl.[5]: **C 07 H 19/01**
A 01 N 43/90

(30) Priorität: **09.09.88 CH 3376/88**

(43) Veröffentlichungstag der Anmeldung:
**14.03.90 Patentblatt 90/11**

(84) Benannte Vertragsstaaten:
**AT BE CH DE ES FR GB GR IT LI LU NL SE**

(71) Anmelder: **Gesellschaft für Biotechnologische Forschung mbH (GBF)**
**Mascheroder Weg 1**
**D-3300 Braunschweig-Stöckheim (DE)**

**CIBA-GEIGY AG**
**Klybeckstrasse 141**
**CH-4002 Basel (CH)**

(72) Erfinder: **Böhlendorf, Bettina**
**Zimmerstrasse 2**
**D-3300 Braunschweig (DE)**

**Bedorf, Norbert, Dr.**
**Krukenbergstrasse 4**
**D-3308 Königslutter (DE)**

**Höfle, Gerhard, Prof. Dr.**
**Alter Weg 12 a**
**D-3300 Braunschweig (DE)**

**Schummer, Dietmar**
**Eichtalstrasse 1**
**D-3300 Braunschweig (DE)**

**Sutter, Marius, Dr.**
**Klingental 5**
**CH-4058 Basel (CH)**

Die Bezeichnung der Erfindung wurde geändert (Richtlinien für die Prüfung im EPA, A-III, 7.3).

## (54) Mikrobizide makrozyklische Laktonderivate.

(57) Derivierte macrocyclische Verbindungen der Formel I

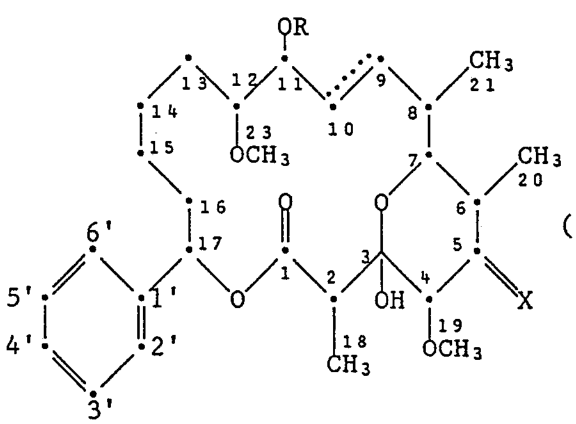

(I)

worin R Wasserstoff, Methyl oder bestimmte Acylgruppen bedeutet, die gepunktete Linie in 9,10-Stellung eine gesättigte Bindung oder im Falle R = Methyl auch eine Doppelbindung ist, und X eine Ketogruppe, oder ein gegebenenfalls substituiertes Oxim, Hydrazon oder Semicarbazon bedeutet, stellen wirksame Mikrobizide zur Bekämpfung von Pflanzenkrankheiten dar. Sie können in üblicher Formulierung als agrarchemische Mittel eingesetzt werden.

EP 0 358 607 A2

**Beschreibung**

## Mikrobizide

Die vorliegende Erfindung betrifft eine macrocyclische Verbindung der Formel I, ein Verfahren zu ihrer Herstellung und ihre Verwendung zur Bekämpfung von Pflanzenkrankheiten sowie pflanzenmikrobizide Mittel, die diese Verbindung als Wirkstoff enthalten.

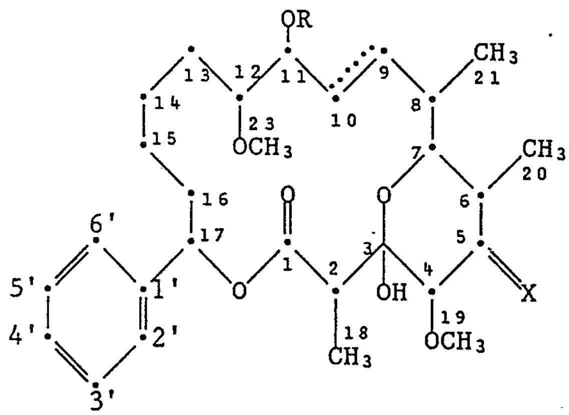

(I)

In dieser Formel bedeutet die gepunktete Linie in 9,10-Stellung wahlweise eine gesättigte Bindung oder eine Doppelbindung, während
R Wasserstoff,$CH_3$ oder -COA ist, worin
A Wasserstoff, $C_3$-$C_6$Cycloalkyl oder unsubstituiertes oder durch Halogen oder $C_1$-$C_3$Alkoxy substituiertes $C_1$-$C_6$Alkyl darstellt, und
X Sauerstoff oder eine der Gruppen =N-OY oder =N-N($R_1$)($R_2$) bedeutet, worin
Y Wasserstoff, $C_1$-$C_6$Alkyl, $C_3$-$C_6$Alkenyl, $C_3$-$C_6$Alkinyl oder eine Acylgruppe -CO-Z darstellt, in der
Z Phenyl, eine durch Halogen oder $C_1$-$C_4$Alkoxy substituierte $C_1$-$C_6$Alkylgruppe oder Wasserstoff, $C_1$-$C_6$Alkyl, $C_2$-$C_6$Alkenyl oder $C_2$-$C_6$Alkinyl bedeutet;
$R_1$ Wasserstoff oder $C_1$-$C_6$Alkyl und
$R_2$ Wasserstoff, $C_1$-$C_6$Alkyl, Phenyl, Carbamoyl($CONH_2$), -COA oder -$SO_2$-$R_3$ bedeuten, worin
$R_3$ $C_1$-$C_6$Alkyl oder unsubstituiertes oder durch $C_1$-$C_4$Alkyl substituiertes Phenyl darstellt;
mit der Massgabe, dass R Methyl bedeutet, wenn in 9,10-Stellung eine Doppelbindung vorliegt.

Die Präparate der Formel I stellen demgemäss 5-Keto-Verbindungen dar oder davon abgeleitete 5-Ketoxime, 5-Hydrazone oder 5-Semicarbazone und gewisse Acylderivate mit Carbonsäuren und Sulfonsäuren.

Unter Alkyl werden je nach Kettenlänge Methyl, Ethyl, Propyl, Butyl, Amyl, Hexyl, sowie ihre Isomeren, z.B. Isopropyl, Isobutyl, sek. Butyl, tert. Butyl, Neopentyl usw. verstanden.

Ein durch Halogen substituierter Alkylrest steht für einen einfach bis perhalogenierten Alkylsubstituenten wie $CHCl_2$, $CH_2Cl$, $CCl_3$, $CF_3$, $C_2F_5$, $CH_2F$, $CH_2Br$, $CH_2CH_2Cl$, CHF-$CH_3$, $CHBr_2$ usw.

Unter Halogen werden Fluor, Chlor, Brom oder Jod verstanden.

Beispiele für Alkylreste, die durch Alkoxy auch im Sinne einer Alkoxyalkoxy-Substitution ein- oder mehrfach substituiert sind, seien -$CH_2OCH_3$, -$CH_2CH_2OCH_3$, -$CH_2CH(CH_3)OCH_3$, -$CH_2OC_2H_5$, -$CH_2OC_3H_7$-i, -$CH_2CH_2CH_2OCH_3$, -$CH_2OCH_2OCH_3$, -$CH_2CH_2OCH_2OCH_3$, -$CH_2OCH_2CH_2OCH_3$, -$CH_2OCH_2OC_2H_5$, -$C(CH_3)_2$-$CH_2OCH_3$, -$CH(CH_3)OCH_2OC_3H_7$-i, -$CH(OCH_3)$-$CH_2OCH_3$ und andere verzweigte oder unverzweigte Reste.

Alkenyl steht für einen aliphatischen Kohlenwasserstoffrest mit einer Doppelbindung, z.B. für Vinyl, Propen(1)-yl, Allyl, Buten(1)-yl, Buten(2)-yl, Hexen(2)-yl usw.

Alkinyl steht für einen aliphatischen Kohlenwasserstoffrest mit einer Dreifachbindung, z.B. für Ethinyl, Propin(1)-yl, Propargyl, Butin(1)-yl, Hexin(5)-yl usw.

$C_3$-$C_6$Cycloalkyl umfasst die Gruppen Cyclopropyl, Cyclobutyl, Cyclopentyl, Cyclohexyl.

Die Erfindung bezieht sich auf Verbindungen der Formel I in allen möglichen stereoisomeren Formen.

Die Verbindungen der Formel I leiten sich von der "Soraphen A" und "Soraphen B" genannten Grundstruktur neuer macrocyclischer Verbindungen der nachstehenden Formel ab

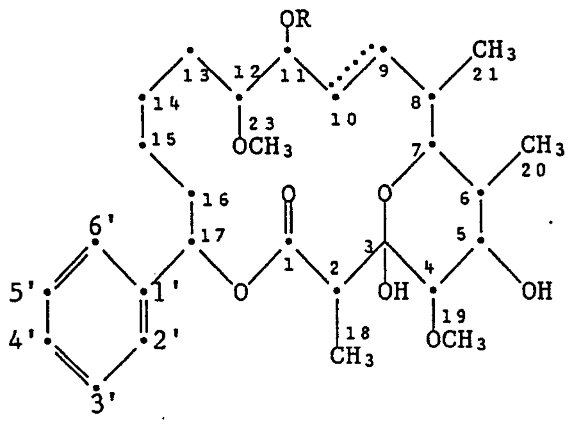

In dieser Formel bedeutet R Methyl, wenn in 9,10-Stellung eine Doppelbindung steht (= Soraphen A) oder R bedeutet Wasserstoff, wenn in 9,10-Stellung eine Einfachbindung steht (= Soraphen B).

Aufgrund der physikochemischen Daten wird angenommen, dass diesen beiden Präparaten folgende Konfiguration zukommt:

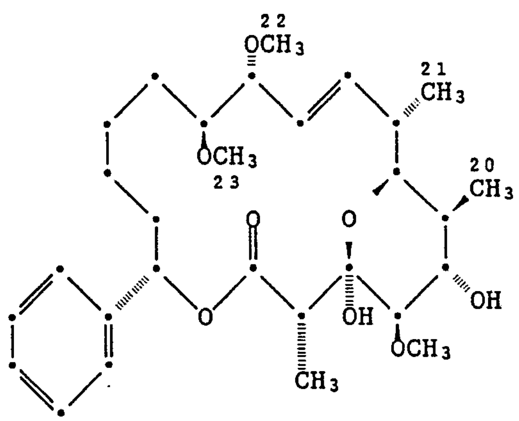

Soraphen A

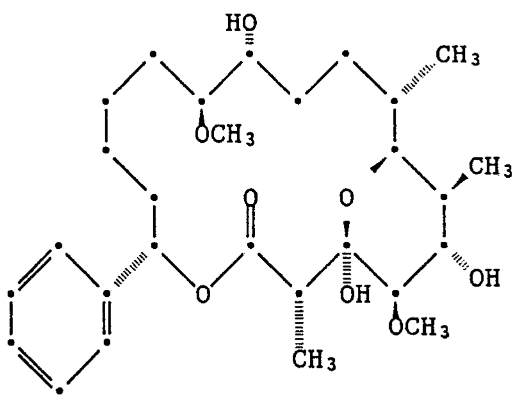

Soraphen B

Soraphen A und B werden durch mikrobiologische Kultivierung eines Sorangium (Polyangium) cellulosum-Stammes "So ce 26" gewonnen. Dieser Stamm wurde am 5. März 1987 bei der "National Collection of Industrial and Marine Bacteria (NCIB)", Torry Research Station, Aberdeen, Grossbritannien, unter der Nummer NCIB 12 411 gemäss Budapester Vertrag hinterlegt. Sorangium cellulosum gehört zur Ordnung der Myxobacterales, Unterordnung Sorangineae, Familie Polyangiaceae.

"So ce 26" selbst oder Mutanten oder Rekombinanten sind Gegenstand der europäischen Patentanmeldung EP-A-0282 455. Der Stamm lässt sich nach üblichen biologischen Methoden, z.B. in Schüttelkulturen oder in Fermentoren mit Nährmedien bei 10-35° C und einem pH-Wert von 6-8, kultivieren. Der Prozess verläuft aerob. Die Bedingungen zur Kultivierung des Mikroorganismus werden per Referenz zur EP-A-0282 455 in die vorliegende Beschreibung eingeführt.

Eine wichtige Untergruppe von Verbindungen der Formel I sind solche, bei denen R Wasserstoff, $CH_3$ oder -COA ist und X Sauerstoff bedeutet.
Diese Gruppe soll hier und im folgenden Untergruppe IA genannt werden.

Unter den Verbindungen der Untergruppe IA stellen solche eine besondere Gruppe dar, bei denen A Wasserstoff, unsubstituiertes oder durch $C_1$-$C_3$ Alkoxy oder ein- oder mehrfach durch Fluor oder Chlor substituiertes $C_1$-$C_3$Alkyl bedeutet. (= Untergruppe IB).

Eine andere wichtige Untergruppe sind jene der Formel I, bei denen R Wasserstoff, $CH_3$ oder -COA ist und X die Gruppe =N-OY darstellt, in der Y Wasserstoff, $C_{1-4}$Alkyl, $C_3$-$C_4$Alkenyl, $C_3$-$C_4$Alkinyl oder -CO-Z bedeutet, und Z eine $C_1$-$C_4$Alkyl-, $C_2$-$C_4$Alkenyl-oder $C_3$-$C_4$Alkinylgruppe oder eine durch Fluor, Chlor oder Methoxy substituierte $C_1$-$C_6$Alkylgruppe ist. (= Untergruppe IC).

Innerhalb der Untergruppe IC sind jene hervorzuheben, bei denen R Wasserstoff, $CH_3$ oder eine Acylgruppe -COA ist, bei der A einen unsubstituierten oder substituierten Rest mit maximal 4 C-Atomen bedeutet. (= Untergruppe ICA).

Eine andere wichtige Gruppe innerhalb der Untergruppe IC sind jene, bei denen R $CH_3$ ist und X die Gruppe -N-OY darstellt, in der Y Wasserstoff, Methyl, Ethyl, Isopropyl, Allyl, Propinyl, Acetyl, Trifluoracetyl, Trichloracetyl oder Methoxyacetyl bedeutet (= Untergruppe ICB).

Eine weitere wichtige Untergruppe sind jene der Formel I, bei denen R Wasserstoff, $CH_3$ oder -COA ist und X die Gruppe =N-N($R_1$)($R_2$) darstellt, bei der $R_1$ Wasserstoff oder $C_1$-$C_4$Alkyl und $R_2$ Wasserstoff, $C_1$-$C_4$Alkyl oder Phenyl sind. (= Untergruppe ID). Unter diesen der Untergruppe ID sind jene hervorzuheben, bei denen R $CH_3$ bedeutet. (= Untergruppe IDA).

Verbindungen der Formel I lassen sich in der 5- bzw. 11-Position, ausgehend vom "Soraphen A" oder "Soraphen B" oder "9, 10-Dihydro-Soraphen A" nach Methoden derivatisieren, die gleichfalls Gegenstand vorliegender Erfindung sind.

Im "Soraphen B" ist die Reaktionsfähigkeit einer Hydroxylgruppe in 5-Stellung verschieden von der in 11-Stellung und lässt sich, gegebenenfalls unter Einsatz von Schutzgruppen, gezielt zur 5-Ketogruppe oxidieren. Weiterhin ist die 3-Hydroxygruppe im "Soraphen A", im Soraphen B" wie auch im "9, 10-Dihydro-Soraphen A" sehr stark abgeschirmt und chemischen Reaktionen schwer zugänglich. "9,10-Dihydro-Soraphen A" wird durch Hydrierung der 9,10-Doppelbindung mit homogenen Katalysatoren auf Basis von Uebergangsmetallkomplexen, z.B. Rhodium- oder Iridium-Kom plexen, aus "Soraphen A" oder einem in 5-Position geschützten Derivat gewonnen. Ein geeigneter Komplex ist z.B. [Iridium(cyclooctadien)(acetonitril) (tricyclohexylphosphin)]tetrafluoroborat.

Diese drei Ausgangsstoffe für die Verbindungen der Formel I sollen hier und im folgenden vereinfachend "Soraphen" genannt werden.

Ein Gegenstand der Erfindung ist ein Verfahren zur Herstellung von Verbindungen der Formel I in allen möglichen stereoisomeren Formen, das dadurch gekennzeichnet ist, dass man bei gegebenenfalls geschützten funktionellen Gruppen in einer macrocyclischen Verbindung der Formel

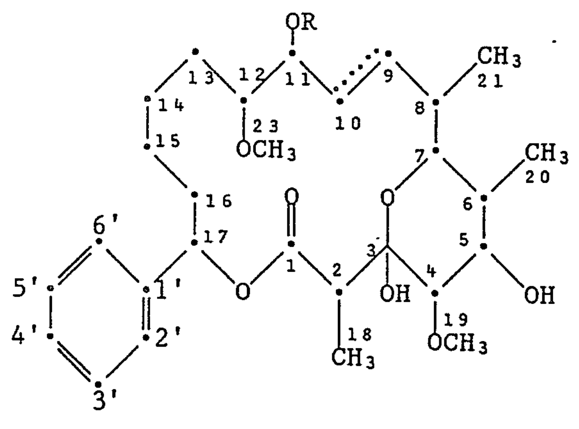

in der die gepunktete Linie in 9,10-Stellung wahlweise eine gesättigte Bindung oder eine Doppelbindung bedeutet mit der Massgabe, dass R Methyl bedeutet, wenn in 9,10-Stellung eine Doppelbindung vorliegt; die OH-Gruppe in 5-Stellung zur Ketogruppe oxidiert, und sofern gewünscht,

a) diese oximiert, und, sofern gewünscht, das Oximderivat durch Einführung des Substituenten Y verethert oder durch Einführung von -CO-Z acyliert, oder

b) die Ketogruppe mit einem Hydrazinderivat

$H_2N$-N($R_1$)($R_2$)

in ein Hydrazon, bzw. wenn $R_2$ Carbamoyl bedeutet, in ein Semicarbazon verwandelt; unter gegebenenfalls weiterer Acylierung und/oder Abspaltung von Schutzgruppen; wobei die genannten Substituenten die für Formel I gegebene Bedeutung haben.

Die 5-Hydroxygruppe des "Soraphens" lässt sich, wie erwähnt, zur 5-Ketogruppe oxidieren. Als Oxidationsmittel kommen beispielsweise Cr-VI-Verbindungen, wie z.B. Pyridiniumdichromat, Pyridiniumchlorochromat, usw., in Frage. Man arbeitet zweckmässigerweise in einem reaktionsinerten Lösungsmittel. Geeignete Lösungsmittel sind z.B. Ether und etherartige Verbindungen wie Dialkylether (Diethylether, Diisopropylether, tert.Butylmethylether, Dimethoxyethan), Dioxan, Tetrahydrofuran (= THF), Anisol, usw.; halogenierte Kohlenwasserstoffe wie Chlorbenzol, Methylenchlorid, Ethylenchlorid, Chloroform, Tetrachlorkohlenstoff, Tetrachlorethylen, usw.; Ketone wie Aceton; Amide wie N,N-Dimethylformamid; Ester wie Ethylacetat, Propylacetat, Butylacetat, usw.; sowie Mischungen dieser Lösungsmittel untereinander oder mit Wasser und/oder anderen üblichen inerten Lösungsmitteln wie Benzol, Xylol, Petrolether, Ligroin, Cyclohexan, usw. In manchen Fällen kann es von Vorteil sein, wenn die Reaktion oder Teilschritte davon unter Schutzgasatmosphäre (z.B. Argon, Helium, Stickstoff, etc.) und/oder in absoluten Lösungsmitteln durchgeführt werden. Die Reaktionstemperatur liegt im Bereich von -50° bis +50°C, vorzugsweise bei -10° bis +30°C.

Oxime und Oximether werden durch Reaktion eines 5-Keto-Soraphens mit einem primären Oxamin der Formel

$H_2N$-OY

oder einem seiner Salze gewonnen, wobei Y die oben angegebene Bedeutung hat, und, sofern Y Wasserstoff darstellt und die Umwandlung in einen Oximether erfolgen soll, durch Weiterreaktion mit einem Halogenid, bevorzugt Chlorid oder Bromid, der Formel

Hal-Y

worin Y $C_1$-$C_6$Alkyl, $C_3$-$C_6$Alkenyl oder $C_3$-$C_6$Alkinyl bedeutet.

Die Herstellung erfolgt durch Reaktion eines 5-Keto-Soraphens mit einem Oxamin bei 10 bis 100°C in einem geeigneten Lösungsmittel, beispielsweise einem Niederalkanol, wie z.B. Methanol, Ethanol, Propanol; einer ether artigen Verbindung, wie z.B. Tetrahydrofuran oder Dioxan; einer aliphatischen Carbonsäure, wie z.B. Essigsäure oder Propionsäure; in Wasser oder in Gemischen dieser Lösungsmittel untereinander oder mit anderen üblichen reaktionsinerten Lösungsmitteln.

Wird das Oxamin in Form eines seiner Salze, z.B. als Hydrochlorid eingesetzt, so ist es vorteilhaft, wenn man zum Abfangen der Säure eine Base zusetzt und zusätzlich in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali-und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetat.

Für die weitere Veretherung sind geeignete Lösungsmittel z.B. Ether und etherartige Verbindungen wie Dialkylether (Diethylether, Diisopropylether, tert.Butylmethylether, Dimethoxyethan, Dioxan, Tetrahydrofuran, Anisol, usw.); halogenierte Kohlenwasserstoffe wie Chlorbenzol, Methylenchlorid, Ethylenchlorid, Chloroform, Tetrachlorkohlenstoff, Tetrachlorethylen, usw.; Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. In manchen Fällen kann es von Vorteil sein, wenn die Reaktionen unter Schutzgasatmosphäre (z.B. Argon, Helium, Stickstoff, etc.) und/oder in absoluten Lösungsmitteln durchgeführt werden. Dei Reaktion verläuft bei 0° bis 100°C, vorzugsweise bei 10° bis 60°C.

Zum Abfangen der als Nebenprodukt gebildeten Säure arbeitet man zweckmässigerweise in Gegenwart eines Neutralisationsmittels. Es kommen z.B. tertiäre Amine wie Trialkylamine (Trimethylamin, Triethylamin, Diisopropylethylamin, Tripropylamin, usw.), Pyridin und Pyridinbasen (4-Dimethylaminopyridin, 4-Pyrrollidylaminopyridin usw.) in Betracht. 5-O-Acylketoxime erhält man aus dem 5-Ketoxim (=N-OH) nach den üblichen Acylierungsmethoden, die auch für die Acylierung eines 11-Hydroxy-Soraphens (R = -COA) anwendbar sind oder für die eines nachfolgend beschriebenen Hydrazons ($R_2$ = -COA bzw. -$SO_3R_3$). Man setzt die entsprechende Carbonsäure oder Sulfonsäure, vorteilhaft im Ueberschuss ein, vorzugsweise aber deren Acylhalogenide, insbesondere Acylbromide oder Acylchloride, im Falle der Carbonsäuren auch deren Acylanhydride.

O-Acylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0°C bis 80°C, bevorzugt bei 10°C bis 50°C ab. Vorzugsweise wird eine organische Base zugegeben. Genannt seien beispielsweise tertiäre Amine wie Triethylamin, Triethylendiamin, Triazol und bevorzugt Pyridin, Imidazol oder 1,8-Diazabicyclo[5.4.0]-undec-7-en (DBU).

Geeignete Lösungsmittel sind z.B.: Ether und etherartige Verbindungen wie Dialkylether (Diethylether, Diisopropylether, tert.-Butylmethylether, Dimethoxiethan, Dioxan, Tetrahydrofuran, Anisol, usw.); halogenierte Kohlenwasserstoffe wie Chlorbenzol, Methylenchlorid, Ethylenchlorid, Chloroform, Tetrachlorkohlenstoff, Tetrachlorethylen, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. In manchen Fällen kann es von Vorteil sein, wenn die Reaktionen unter Schutzgasatmosphäre (z.B. Argon, Helium, Stickstoff, etc.) und/oder in absoluten Lösungsmitteln durchgeführt werden.

Setzt man zur Acylierung eine freie Carbonsäure oder Sulfonsäure als Reaktand ein, so wird diese Reaktion zweckmässigerweise in Gegenwart wasserabspaltender Reagenzien durchgeführt. Man arbeitet beispielsweise in Anwesenheit von Dicyclohexylcarbodiimid und Pyridin oder in Gegenwart von Azodicarbonsäuredialkylester und Triphenylphosphin.

Werden Säurehalogenide oder Säureanhydride zur Acylierung eingesetzt, so erweist sich der Zusatz eines

Neutralisationsmittels als vorteilhaft. tertiäre Amine wie Trialkylamine, Pyridin oder Pyridinbasen wie 4-Dimethylaminopyridin sind zweckmässige Reagenzien, die zum Teil auch als Lösungsmittel dienen können.

5-Hydrazon-Derivate der Formel I lassen sich aus 5-Keto-Soraphen durch Reaktion mit einem Hydrazin-Derivat

$H_2N-N(R_1)(R_2)$

oder einem seiner Salze mit anorganischen oder organischen Säuren darstellen, wobei in Gegenwart einer Base wie CaO, Trialkylamin, Na-Acetat, Pyridin oder zur Säurekatalyse in Gegenwart einer Säure wie Essigsäure, Chlorwasserstoffsäure oder Schwefelsäure gearbeitet wird. Die Reaktionstemperatur beträgt 0° bis 100° C. Als Lösungsmittel kommen die obengenannten, bevorzugt Wasser, Alkohol, Ether, Dioxan, Benzol oder Eisessig in Frage.

Sind im Molekül oder im Reaktanden störende funktionelle Gruppen wie OH, $NH_2$, -COOH, so können diese, wie bereits oben erwähnt, durch Acetylierung oder Einführung anderer Schutzgruppen einleitend maskiert werden [T.W. Green "Protective Groups in Organic Synthesis", J. Wiley & Sons, 1981 (New York)].

Die Aufzählung aller vorgenannten Methoden ist nicht limitierend. Gewünschtenfalls können die Endprodukte auf übliche Art und Weise gereinigt werden, z.B. durch Waschen, Digerieren, Extraktion, Umkristallisation, Chromatographie usw..

Die beschriebenen Herstellungsverfahren einschliesslich aller Teilschritte sind ein Bestandteil der vorliegenden Erfindung.

Es ist zu beachten, dass die macrocyclischen Soraphene der Formel I normalerweise in der angegebenen Hemiacetalform vorliegen, diese Form jedoch eine reversible Ringöffnung nach dem Schema

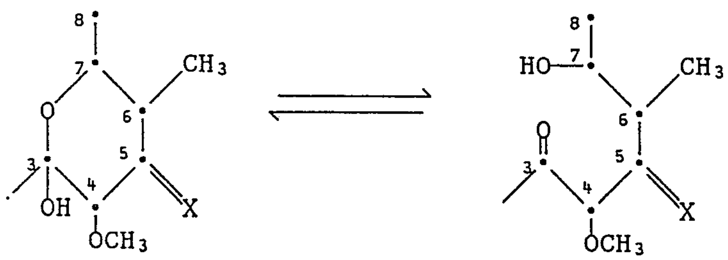

erfahren kann. Je nach Herstellungs- bzw. nach Aufarbeitungsmethodik fallen, abhängig vom pH-Wert und vom Lösungsmittel, die Soraphene, in der einen oder anderen Form oder als Gemisch beider Formen an. Charakteristisch für die Ringöffnung ist die Verschiebung des $^{13}C$-NMR-Signals in der 3-Position und die der $^{1}H$-NMR-Signale in bestimmten anderen Positionen. Beim Soraphen A werden beispielsweise folgende Veränderungen beobachtet: $^{13}C$-NMR($CDCl_3$, δ in ppm) 99,5 → 203,1(3-C).$^{1}H$-NMR($CDCl_3$, δ in ppm): 3,14→3,72(2-H); 3,18→4,5(4-H); 3,83→3,16 (7-H); 5,86→5,7 (17-H). Aehnliche Verschiebungen werden auch bei den hierin beschriebenen Soraphen-Derivaten der Formel I beobachtet. Die Formel I vorliegender Erfindung umfasst grundsätzlich sowohl die bei niedrigen pH-Werten bevorzugte 3-Hemiacetalform wie auch geöffnete 3-Keto-7-hydroxyform.

Es wurde gefunden, dass Verbindungen der Formel I ein für praktische Bedürfnisse sehr günstiges biozides Spektrum gegen phytopathogene Mikroorganismen, insbesondere gegen Pilze, aufweisen. Sie besitzen sehr vorteilhafte kurative, systemische und insbesondere präventive Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Als Mikrobizide sind die Wirkstoffe der Formel I z.B. gegen die den folgenden Klassen angehörenden phytopathogenen Pilze wirksam: Fungi imperfecti (z.B. insbesondere Botrytis, ferner Pyricularia, Helminthosporium, Fusarium, Septoria, Cercospora und Alternaria); Basidio myceten (z.B. Rhizoctonia, Hemileia, Puccinia). Darüber hinaus wirken sie gegen die Klasse der Ascomyceten (z.B. insbesondere Venturia und Erysiphe, ferner Podosphaera, Monilinia, Uncinula) und der Oomyceten (z.B. Phytophthora, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I in allen möglichen stereoisomeren Formen enthalten, insbesondere pflanzenschützende Mittel, sowie deren Verwendung auf dem Agrarsektor oder verwandten Gebieten.

Eingeschlossen ist auch ein Verfahren zur Behandlung von Pflanzen, das sich durch Applikation der neuen Verbindungen der Formel I bzw. der entsprechenden neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute);

Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Ananas, Zuckerrohr, Tee, Pfeffer, Weinreben, Hopfen, Bananen- und Naturkautschukgewächse sowie Zierpflanzen (Compositen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Verbindungen der Formel I können auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 500 g Aktivsubstanz (AS) pro Hektar, bevorzugt bei 50 g bis 200 g AS/ha.

Die Formulierungen d.h. die den Wirkstoff der Formel I und einen festen oder flüssigen Zusatzstoff enthaltenden Mittel werden in bekannter Weise hergestellt.

Als Lösungsmittel kommen in Frage: Aromatische und aliphatische Kohlenwasserstoffe, wie z.B. Xylolgemische, Cyclohexan oder Paraffine; dann Alkohole und Glykole sowie deren Ether und Ester, wie Ethanol, Ethylenglykol, Ethylenglykolmonomethyl- oder -ethylether, Essigsäureester; Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein. Häufiger werden jedoch sog. synthetische Tenside verwendet, insbesondere Alkansulfonate, Fettalkoholsulfate, sulfonierte Benzimidazolderivate oder Alkylsulfonate.

Als nichtionische Tenside kommen Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Als weitere Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 95 % Wirkstoff der Formel I, 99,9 bis 5 %

eines festen oder flüssigen Zusatzstoffes und 0 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken. (Es bedeuten: h = Stunde. PSC = präparative Schicht-Chromatographie DC = Dünnschichtchromatographie. RT = Raumtemperatur).

## 1. Herstellungsbeispiele

### H-1. Herstellung von Soraphen A-5-on (Verb. Nr. 1)

500 mg (0.84 mmol) Soraphen A-Etherat (M = 594.87) werden in 5 ml Dichlormethan gelöst und mit 300 mg (1:39 mmol) Pyridiniumchlorochromat versetzt. Man lässt 24 Stunden bei Raumtemperatur rühren und filtriert dann über Kieselgel 60 mit $CH_2Cl_2$/Aceton 95:5. Man erhält 375 mg (0.72 mmol, 86 %) des Produktes als blassgrünes Oel. Zur Charakterisierung kann das Rohprodukt mittels PSC (Merck, Kieselgel 60, Laufmittel: $CH_2Cl_2$/Aceton 95:5) gereinigt werden.

In gleicher Weise lässt sich 9,10-Dihydro-Soraphen A zur entsprechenden 5-Oxo-Verbindung Nr. 2 (in Tabelle 3) oxydieren.

### H-2. Herstellung von Soraphen A-5-hydroxyimin (Verb. Nr. 15)

60 mg (0.115 mmol) Soraphen A-5-on werden in 1.5 ml Pyridin gelöst und mit 32 mg (0.461 mmol) Hydroxylaminhydrochlorid versetzt. Man lässt 45 Minuten bei Raumtemperatur rühren und versetzt dann mit Ethylacetat und halbkonzentrierter HCl. Die organische Phase wird nacheinander mit 5 %iger $NaHCO_3$-Lösung und gesättigter NaCl-Lösung gewaschen, über $NaSO_4$ getrocknet und am Rotationsverdampfer eingeengt. Man erhält 56 mg des Rohproduktes, das mittels PSC (Kieselgel 60), Lm: $CH_2Cl_2/Et_2O$ 60:40, Elution der unpolaren Zone) gereinigt wird.
Ausbeute: 17.4 mg (0.033 mmol, 29 %) farbloses Oel.

### H-3. Herstellung von Soraphen A-5-semicarbazid (Verb. Nr. 40)

50 mg (0,096 mmol) Soraphen A-5-on werden in 1 ml Ethanol gelöst und mit 16 µl (16 mg, 0,20 mmol) Pyridin und 13 mg (0,116 mmol) Semicarbazid Hydrochlorid versetzt. Nach 30minütigem Rühren bei Raumtemperatur wird die Reaktionsmischung weitgehend eingeengt, mit 1N-HCl versetzt und mit Ethylacetat extrahiert. Die vereinigten organischen Phasen werden mit 5%iger $NaHCO_3$-Lösung und konz. NaCl-Lösung gewaschen, über $Na_2SO_4$ getrocknet und einrotiert. Es werden 46 mg des Rohproduktes erhalten, das mittels PSC (Merck, Kieselgel 60; Lm: Dichlormethan/Aceton/Methanol 80:20:2; Eluieren der unpolaren Zone) gereinigt wird.
Ausbeute: 16 mg (0,028 mmol, 29 %).

Tabelle 1

| Verb. Nr. | MS(EI) | | $^1$H-NMR-Daten ausgewählter Signale ($CDCl_3$, δ in ppm) | | | | |
|---|---|---|---|---|---|---|---|
| | $R_f$(Lm)* | $M^+$ | 2-H | 4-H | 6-H | 3-OH | R,X |
| 15 | 0.29 (1) | 533 | 3.25 | ? | 1.67 | 4.01 | 7.72 (b,N-OH) |
| 1 | 0.77 (1) | 518 | 3.23 | 3.18 | 2.56 | 4.01 | - |
| 17 | | 547 | 3.16 | 3.20 | 1.66 | 4.02 | 3.92 (s, 3H) |
| 40 | | 575 | 3.24 | 3.26 | 2.95 | ? | - |
| 35 | 0.39 (1) | 532 | 3.24 | 3.45 | 2.86 | 4.01 | 5.4 (2H,b,$NH_2$) |
| 36 | 0.49 (3) | 560 | 3.26 | 3.41 | ? | 3.91 | 2.51 (6H,-$N(CH_3)_2$) |
| 42 | 0.31 (1) | | 3.16 | 3.35 | 2.70 | 3.89 | 2.42 (s, 3H)(p-Tolyl) |

* Dichlormethan/Aceton (1) = 90:10

Dichlormethan/Aceton (3) = 75:25

Tabelle 2

| Verb. Nr. | $^{13}C$-NMR-Daten ausgewählter Signale ($CDCl_3$, δ in ppm) C-4/C-7/C-17 | C-5,R,X |
|---|---|---|
| 15 | 75.7/75.4/77.6* | 156.8 |
| 1 | 74.8/75.7/82.4* | 207.2 |
| 17 | 74.7/75.4/77.4* | 61.9,155.4 |
| .40 | 74.8/75.1/79.8* | 148.2,157.8 |
| 35 | 74.6/75.1/80.1* | 148.5 |
| 36 | 74.6/76.7/80.0* | 165.8,48.2 |

* Zuordnung offen

H-4. Herstellung von 5-(Acetoxy-imino)-Soraphen A (Verb. Nr. 49)

40 mg (77 µmol) des gemäss H-2 gewonnenen Soraphen A-5-hydroxyimin werden in 1 ml Aceton gelöst und mit 50 mg Kaliumcarbonat und 12 mg (2 eq) Acetylchlorid versetzt. Nach 3 Stunden Rühren bei RT zeigt ein DC (Laufmittel Dichlormethan/Aceton, 9:1 v/v; Edukt Rf 0.4, Produkt Rf 0.7) nahezu vollständige Umsetzung an. Die Lösung wird mit 10 ml gesättigter Ammoniumchloridlösung verdünnt (pH 8), zweimal mit Ethylacetat extrahiert, das Lösungsmittel abdestilliert und das Produkt mit Hilfe der PSC gereinigt (Kieselgel Si 60, 1 mm, Laufmittel Dichlormethan/Aceton, 9:1, v/v; Rf 0.6).
Ausbeute: 18.3 mg = 41 % der Theorie.
$^{1}H$-NMR ($CDCl_3$): δ = 2.55 (m, 1 H, H-8); 3.24 (g, 1 H, H-2); 3.40 (m, 1 H, H-6); 3.68 (s, 1 H, H-4); 2.21 (s, 3 H, Acetyl).
$^{13}C$-NMR ($CDCl_3$): δ =31.06 d, 34.91 d (C-6, C-8); 98.99 s (C-3); 163.35 s (C-5); 170.50 s (C-1); 19.68 q, 168.49 s (Acetyl).
IR (Film): ν = 3525, 2933, 2869, 2829, 1772, 1735, 1481, 1376, 1280, 1233, 1195, 1091, 1033, 985, 919, 857, 759, 701 $cm^{-1}$.
UV (Methanol): λmax (1g ε) = 202 nm (4.30).
MS (70 eV): m/e (%) = 575 [4(M-H)$^+$], 543 (3), 516 (2), 484 (3), 386 (12), 259 (14), 210 (14), 208 (14), 189 (38), 157 (91), 71 (100).

| Hochauflösung: $C_{31}H_{45}NO_9$ | Ber.: | 575.3094 |
|---|---|---|
| | Gef.: | 575.3100 (M-1)$^+$. |

H-5. Herstellung von 5-(Benzoyloxy-imino)-Soraphen A (Verb. Nr. 44)

40 mg (77 µmol) des gemäss H-2 gewonnenen Produkts werden in 1 ml Aceton gelöst und mit 50 mg Kaliumcarbonat und 28 mg (2 eq) Benzoylchlorid versetzt. Nach 3 Stunden Rühren bei RT zeigt ein DC (Laufmittel Dichlormethan/Aceton, 9:1, v/v; Edukt Rf 0.4, Produkt Rf 0.8) nahezu vollständige Umsetzung an. Die Lösung wird mit 10 ml gesättigter Ammoniumchloridlösung verdünnt (pH 8), zweimal mit Ethylacetat extrahiert, das Lösungsmittel abdestilliert und das Produkt mit Hilfe der PSC gereinigt (Kieselgel Si 60, 1 mm, Laufmittel Dichlormethan/Aceton, 9:1, v/v; Rf 0.65).
Ausbeute: 24.0 mg = 49 % der Theorie.
$^{1}H$-NMR ($CDCl_3$): δ = 2.59 (m, 1 H, H-8); 3.24 (q, 1 H, H-2); 3.27 (s, 1 H, H-4); 3.54 (m, 1 H, H-6); 3.80 (s, 3-OH); 7.49, 7.61, 8.03 (Benzoyl).
$^{13}C$-NMR ($CDCl_3$): δ =31.37 d, 34.88 d (C-6, C-8); 99.04 s (C-3); 163.76 s (C-5); 170.62 s (C-1); 128.72 d, 128.89 s, 129.65 d, 133.56 d, 164.61 s (Benzoyl).
IR (Film): ν = 3527, 2960, 2933, 2871, 1737, 1481, 1280, 1239, 1181, 1129, 1087, 1021, 985, 688 $cm^{-1}$.
UV (Methanol): λmax (1 g ε) = 235 nm (4.25).
MS (70 eV): m/e (%) = 637 [1(M-H)$^+$], 605 (1), 517 (2), 484 (3), 321 (4), 210 (14), 208 (11), 189 (35), 157 (98), 105 (100), 71 (90).

| Hochauflösung: $C_{36}H_{47}NO_9$ | Ber.: | 637.3251 |
|---|---|---|
| | Gef.: | 637.3262 (M-1)$^+$. |

H-6. Herstellung von 5-(t-Butoxy-imino)-Soraphen A (Verb. Nr. 18)

52 mg (0.1 mmol) des gemäss Beispiel H-1 gewonnenen Soraphen A-5-on werden in 1 ml Pyridin gelöst und mit 20 mg O-t-Butylhydroxylaminhydrochlorid (1.5 eq) versetzt. Nach 30minütigem Rühren bei 60°C zeigt ein DC (Laufmittel Dichlormethan/Aceton, 9:1, v/v; Edukt Rf 0.65, Produkt Rf 0.7) eine vollständige Reaktion an.

Die Lösung wird mit 10 ml pH 5 Puffer verdünnt, zweimal mit Ethylacetat extrahiert, das Lösungsmittel wird abdestilliert und das Produkt mit Hilfe der PSC gereinigt (Kieselgel Si 60, 1 mm, Laufmittel Dichlormethan/Aceton, 9:1, v/v; Rf 0.7).
Ausbeute: 40.7 mg = 69 % der Theorie.
$^1$H-NMR ($CDCl_3$): δ = 2.53 (m, 1 H, H-8); 3.24 (q, 1 H, H-2); 3.39 (m, 1 H, H-6); 3.41 (s, 1 H, H-4); 3.93 (s, 3-OH); 1.30, (s, 9 H, t-Butyl).
$^{13}$C-NMR ($CDCl_3$): δ =29.10 d, 35.08 d (C-6, C-8); 98.80 s (C-3); 153.22 s (C-5); 170.99 s (C-1); 27.51 q, 78.22 s (t-Butyl).
IR (Film): ν = 3533, 2968, 2935, 2875, 2827, 1737, 1481, 1370, 1274, 1232, 1191, 1091, 1033, 977, 948, 861, 759, 699 $cm^{-1}$.
UV (Methanol): λmax (1 g ε) = 206 nm (4.35).
MS (70 eV): m/e (%) = 589 [1(M-H)$^+$], 557 (1), 312 (1), 279 (29), 167 (71), 149 (100), 129 (60), 71 (77), 57 (90).

| | | |
|---|---|---|
| Hochauflösung: $C_{33}H_{51}NO_8$ | Ber.: | 589.3614 |
| | Gef.: | 589.3620 $(M-1)^+$. |

H-7. Herstellung von 5-(Allyloxy-imino)-Soraphen A (Verb. Nr. 20)

52 mg (0.1 mmol) des gemäss H-1 gewonnenen Produkts werden in 1 ml Pyridin gelöst und mit 22 mg O-Allylhydroxylaminhydrochlorid (2 eq) versetzt. Nach 30minütigem Rühren bei 60°C zeigt ein DC (Laufmittel Dichlormethan/Aceton, 9:1, v/v; Edukt Rf 0.65, Produkt Rf 0.7) eine vollständige Reaktion an. Die Lösung wird mit 10 ml 1 N HCl verdünnt, zweimal mit Ethylacetat extrahiert, das Lösungsmittel wird abdestilliert und das Produkt mit Hilfe der PSC gereinigt (Kieselgel Si 60, 1 mm, Laufmittel Dichlormethan/Aceton, 9:1, v/v; Rf 0.7).
Ausbeute: 31.2 mg = 55 % der Theorie.
$^1$H-NMR ($CDCl_3$): δ = 2.53 (m, 1 H, H-8); 3.24 (q, 1 H, H-2); 3.32 (d, 1 H, H-4); 3.45 (m, 1 H, H-6); 3.99 (s, 3-OH); 4.62, 5.20, 5.28, 5.98 (Allyl).
$^{13}$C-NMR ($CDCl_3$): δ =29.49 d, 35.01 d (C-6, C-8); 98.86 s (C-3); 155.60 s (C-5); 170.83 s (C-1); 74.76 t, 117.18 t, 134.35 d (Allyl).
IR (Film): ν = 3531, 2960, 2933, 2863, 2827, 1735, 1463, 1382, 1274, 1232, 1189, 1129, 1091, 1031, 968 $cm^{-1}$.
UV (Methanol): λmax (1 g ε) = 205 nm (4.27).
MS (70 eV): m/e (%) = 574 (8, M$^+$), 542 (8), 352 (5), 310 (5), 286 (11), 266 (14), 257 (15), 189 (18), 157 (75), 91 (95), 71 (100).

| | | |
|---|---|---|
| Hochauflösung: $C_{32}H_{47}NO_8$ | Ber.: | 573.3301 |
| | Gef.: | 573.3312 $(M-1)^+$. |

H-8. Herstellung von 5-(Methoxyacetoxy-imino)-Soraphen A (Verb. Nr. 12)

40 mg (77 µmol) des gemäss H-2 gewonnenen Produkts werden in 1 ml Aceton gelöst und mit 50 mg Kaliumcarbonat und 22 mg (3 eq) Methoxyacetylchlorid versetzt. Nach 3 Stunden Rühren bei RT zeigt ein DC (Laufmittel Dichlormethan/Aceton, 9:1, v/v; Edukt Rf 0.4, Produkt Rf 0.6) nahezu vollständige Umsetzung an. Die Lösung wird mit 10 ml gesättigte Ammoniumchloridlösung verdünnt (pH 8), zweimal mit Ethylacetat extrahiert, das Lösungsmittel abdestilliert und das Produkt mit Hilfe der PSC gereinigt (Kieselgel Si 60, 1 mm, Laufmittel Dichlormethan/Aceton, 9:1, v/v; Rf 0.5).
Ausbeute: 16.5 mg = 35 % der Theorie.
$^1$H-NMR ($CDCl_3$): δ = 2.56 (m, 1 H, H-8); 3.24 (q, 1 H, H-2); 3.44 (m, 1 H, H-6); 3.67 (s, 1 H, H-4); 4.07 (s, 3-OH); 3.27, 4.25 (Methoxyacetyl).
$^{13}$C-NMR ($CDCl_3$): δ =31.06 d, 34.85 d (C-6, C-8); 98.94 s (C-3); 164.34 s (C-5); 170.47 s (C-1); 56.28 q, 69.14 t, 168.37 s (Methoxyacetyl).
IR (Film): ν = 3523, 2960, 2933, 2875, 2863, 1789, 1735, 1463, 1286, 1276, 1232, 1122, 1091, 988 $cm^{-1}$.
UV (Methanol): λmax (1 g ε) = 223 nm (4.79).
MS (70 eV): m/e (%) = 606 (1, M$^+$), 518 (1), 502 (1), 485 (1), 417 (3), 210 (8), 189 (20), 164 (11), 157 (82), 91 (89), 45 (100).

| | | |
|---|---|---|
| Hochauflösung: $C_{32}H_{47}NO_{10}$ | Ber.: | 605.3200 |
| | Gef.: | 605.3201 $(M-1)^+$. |

Auf diese Art oder nach einer der weiter oben angegebenen Methoden werden folgende Verbindungen der Formel I erhalten.

Tabelle 3

| Nr. | R | 9,10-Stellung | X |
|---|---|---|---|
| 1 | $CH_3$ | DB | O |
| 2 | $CH_3$ | – | O |
| 3 | H | – | O |
| 4 | CHO | – | O |
| 5 | $COCH_3$ | – | O |
| 6 | $COCCl_3$ | – | O |
| 7 | CO-$C_6H_{13}$-n | – | O |
| 8 | CO-Cyclopropyl | – | O |
| 9 | CO-Cyclohexyl | – | O |
| 10 | CO-$CH_2OCH_3$ | – | O |
| 11 | $COC_2F_5$ | – | O |
| 12 | $CH_3$ | DB | N-O-CO-$CH_2$-$OCH_3$ |
| 13 | $COCH_2$-$OCH_3$ | – | O |
| 14 | H | – | N-OH |
| 15 | $CH_3$ | DB | N-OH |
| 16 | $CH_3$ | – | N-OH |
| 17 | $CH_3$ | DB | N-$OCH_3$ |
| 18 | $CH_3$ | DB | N-O-tert.$C_4H_9$ |
| 19 | $CH_3$ | – | N-$OC_6H_{13}$-n |
| 20 | $CH_3$ | DB | N-$OCH_2$-CH=$CH_2$ |
| 21 | $CH_3$ | DB | N-O$(CH_2)_4$-CH=$CH_2$ |
| 22 | $CH_3$ | DB | N-O-$CH_2$-C≡CH |
| 23 | H | – | N-O$(CH_2)_4$-C≡CH |
| 24 | $CH_3$ | DB | N-O-CHO |
| 25 | $CH_3$ | – | N-O-CHO |
| 26 | H | – | N-OCO-$CH_3$ |
| 27 | H | – | N-OCO-$CF_3$ |
| 28 | $COCH_3$ | – | N-OCO-$CH_3$ |
| 29 | CHO | – | N-OCHO |
| 30 | $CO(CH_2)_5I$ | – | N-OH |
| 31 | $CO(CH_2)_5$-$OC_3H_7$ | – | N-$OCH_3$ |

Tabelle 3 (Fortsetzung)

| Nr. | R | 9,10-Stellung | X |
|---|---|---|---|
| 32 | CO-Cyclopropyl | - | $N-OCO-C_6H_{13}$ |
| 33 | CO-Cyclohexyl | - | N-OH |
| 34 | $CH_3$ | DB | $N-OCO-C_4H_9$ |
| 35 | $CH_3$ | DB | $N-NH_2$ |
| 36 | $CH_3$ | DB | $N-N(CH_3)_2$ |
| 37 | $CH_3$ | DB | $N-NH-CH_3$ |
| 38 | $CH_3$ | DB | $N-NH-C_6H_{13}-n$ |
| 39 | $CH_3$ | DB | $N-NH-C_6H_5$ |
| 40 | $CH_3$ | DB | $N-NH-CO-NH_2$ |
| 41 | $CH_3$ | DB | N-NH-CHO |
| 42 | $CH_3$ | DB | $N-NH-SO_2-$ 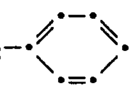 $-CH_3$ |
| 43 | $CH_3$ | DB | $N-NH-CO-C_6H_{13}-n$ |
| 44 | $CH_3$ | DB | $N-O-CO-C_6H_5$ |
| 45 | $CH_3$ | - | $N-O-CO-C_6H_5$ |
| 46 | $-CO-tert.C_4H_9$ | - | O |
| 47 | $CH_3$ | DB | $N-O-C_3H_7(iso)$ |
| 48 | $CH_3$ | DB | $N-NH-C_3H_7(iso)$ |
| 49 | $CH_3$ | DB | $N-O-CO-CH_3$ |

2. Formulierungsbeispiele für den Wirkstoff der Formel I (% = Gewichtsprozent) ["Wirkstoff" bedeutet im folgenden einen Wirkstoff aus vorstehender Tabelle 3]

| 2.1 Emulsions-Konzentrate | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3 Granulate | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel. Diese lassen sich durch weiteren Zusatz der drei Trägerstoffe auf anwendungsfertige Stäube mit 0,001 % Wirkstoff vermahlen.

| 2.5 Spritzpulver | a) | b) | c) |
|---|---|---|---|
| Wirkstoff | 25 % | 50 % | 75 % |
| Na-Ligninsulfonat | 5 % | 5 % | - % |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - % |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Umhüllungs-Granulat

| | |
|---|---|
| Wirkstoff | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.7 Suspensions-Konzentrat

| | |
|---|---|
| Wirkstoff | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele an Pflanzen

(Im folgenden bedeutet "Wirkstoff" ohne weiteren Hinweis ein Präparat aus der Tabelle 3.)

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

Der Pilzbefall wurde in beiden Versuchen vollständig durch den Wirkstoff gehemmt.

Unbehandelte aber infizierte Kontrollpflanzen wiesen dagegen einen Puccinia-Befall von 100 % auf.

Beispiel 3.2: Wirkung gegen Phytophthora auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luchtfeuchtigkeit und 20° C.

b) Systemische Wirkung

Zu Tomatenpflanzen wurde nach 3-wöchiger Anzucht eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des

Pilzbefalls erfolgte nach einer Inkubation der infizierten Pflanzen während 5 Tagen bei 90-100 % relativer Luftfeuchtigkeit und 20°C.

In beiden Versuchen wurde bei der Auswertung kein Pilzbefall beobachtet.

Beispiel 3.3: Wirkung gegen Plasmopara viticola auf Reben

Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

Im Gegensatz zu den unbehandelten, aber infizierten Kontrollpflanzen mit 100 % Pilzbefall waren die mit dem Wirkstoff I behandelten Pflanzen befallsfrei.

Beispiel 3.4: Wirkung gegen Cercospora arachidicola auf Erdnusspflanzen

Residual protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einem Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Die mit dem Wirkstoff I behandelten Pflanzen wiesen geringen Befall auf, die mit einer der Verbindungen Nr. 1, 2, 12, 13, 15, 17, 18, 20, 35, 36, 40, 42, 44, 49 behandelten Pflanzen waren ohne Befall. Unbehandelte aber infizierte Kontrollpflanzen wiesen dagegen einen Cercospora-Befall von 100 % auf. Verbindung Nr. 12 wies noch in einer Verdünnung von 0,006 % vollständige Hemmung des Pilzbefalls auf (0-5 % Befall).

Beispiel 3.5: Wirkung gegen Venturia inaequalis auf Apfeltrieben

Residual-protektive Wirkung

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Die mit dem Wirkstoff I behandelten Stecklinge waren befallsfrei.

Beispiel 3.6: Wirkung gegen Botrytis cinerea an Apfelfrüchten

Residual-protektive Wirkung

Künstlich verletzte Aepfel werden behandelt, indem eine aus Spritzpulver der Wirksubstanz hergestellte Spritzbrühe (0,02 % Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Die Verbindungen Nr. 1,2, 12, 13, 15, 17, 18, 20, 35, 36, 40, 42, 44 und 49 hemmten den Pilzwuchs vollständig (0-5 % Befall), während die Fäulnis sich über die Kontrollfrüchte ausgebreitet hatte.

Beispiel 3.7: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Die Pflanzen waren in beiden Versuchen befallsfrei, die Kontrollpflanzen vollständig befallen.

Beispiel 3.8: Wirkung gegen Rhizoctonia solani (Bodenpilz auf Reispflanzen)

Protektiv-lokale Bodenapplikation

12 Tage alte Reispflanzen werden mit einer aus einer Formulierung des Wirkstoffes hergestellten Spritzbrühe (0,006 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27°C (Tag), bzw. 23°C (Nacht) und 100 % rel. Luftfeuchtigkeit (Feuchtkasten) in der Klimakammer wird der Pilzbefall auf Blattscheide, Blättern und Stengel beurteilt.

Nach Behandlung mit dem Wirkstoff trat kein Befall auf.

**Patentansprüche**

1. Macrocyclische Verbindung der Formel I

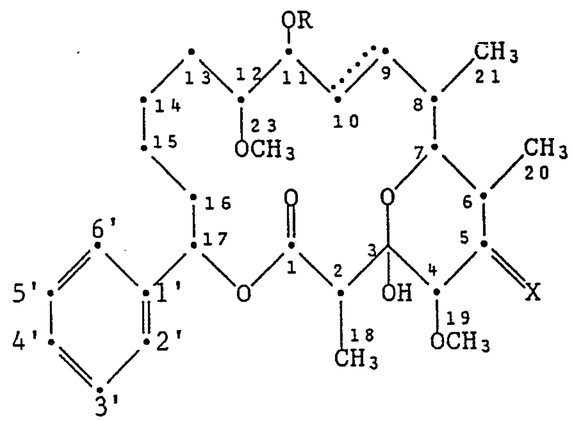

(I)

in der die gepunktete Linie in 9,10-Stellung wahlweise eine gesättigte Bindung oder eine Doppelbindung bedeutet, während

R Wasserstoff, $CH_3$ oder -COA ist, worin

A Wasserstoff, $C_3$-$C_6$Cycloalkyl, unsubstituiertes oder durch Halogen oder $C_1$-$C_3$Alkoxy substituiertes $C_1$-$C_6$Alkyl darstellt,

X Sauerstoff oder eine der Gruppen =N-OY oder =N-N($R_1$)($R_2$) bedeutet, worin

Y Wasserstoff, $C_1$-$C_6$Alkyl, $C_3$-$C_6$Alkenyl, $C_3$-$C_6$Alkinyl oder eine Acylgruppe -CO-Z darstellt, in der

Z Phenyl, eine durch Halogen oder $C_1$-$C_4$Alkoxy substituierte $C_1$-$C_6$Alkylgruppe oder Wasserstoff, $C_1$-$C_6$Alkyl, $C_2$-$C_6$Alkenyl oder $C_2$-$C_6$Alkinyl bedeutet;

$R_1$ Wasserstoff oder $C_1$-$C_6$Alkyl und

$R_2$ Wasserstoff, $C_1$-$C_6$Alkyl, Phenyl, Carbamoyl($CONH_2$), -COA oder -$SO_2$-$R_3$ bedeuten, worin

$R_3$ $C_1$-$C_6$Alkyl oder unsubstituiertes oder durch $C_1$-$C_4$Alkyl substituiertes Phenyl darstellt;

mit der Massgabe, dass R Methyl bedeutet, wenn in 9,10-Stellung eine Doppelbindung vorliegt.

2. Eine Verbindung der Formel I gemäss Anspruch 1, worin R Wasserstoff, $CH_3$ oder -COA ist und X Sauerstoff bedeutet.

3. Eine Verbindung gemäss Anspruch 2, worin A Wasserstoff unsubstituiertes oder durch $C_1$-$C_3$-Alkoxy oder ein- oder mehrfach durch Fluor oder Chlor substituiertes $C_1$-$C_3$-Alkyl bedeutet.

4. Eine Verbindung gemäss Anspruch 1, worin R Wasserstoff, $CH_3$ oder -COA ist und X die Gruppe =N-OY darstellt, in der Y Wasserstoff, $C_1$-$C_4$Alkyl, $C_3$-$C_4$Alkenyl, $C_3$-$C_4$Alkinyl oder -CO-Z bedeutet, und Z eine $C_1$-$C_4$Alkyl-, $C_2$-$C_4$Alkenyl-oder $C_3$-$C_4$Alkinylgruppe oder eine durch Fluor, Chlor oder Methoxy substituierte $C_1$-$C_6$Alkylgruppe ist.

5. Eine Verbindung gemäss Anspruch 4, worin R Wasserstoff, $CH_3$ oder eine Acylgruppe -COA ist, bei der A einen unsubstituierten oder durch Halogen oder Alkoxy substituierten Alkylrest mit maximal 4 C-Atomen bedeutet.

6. Eine Verbindung gemäss Anspruch 4, worin R Methyl ist und X die Gruppe =N-O-Y darstellt, in der Y Wasserstoff, Methyl, Ethyl, Isopropyl, Allyl, Propinyl, Acetyl, Trifluoracetyl, Trichloracetyl oder Methoxyacetyl bedeutet.

7. Eine Verbindung gemäss Anspruch 1, worin R Wasserstoff, $CH_3$ oder -COA ist und X die Gruppe =N-N($R_1$)($R_2$) darstellt, bei der $R_1$ Wasserstoff oder $C_1$-$C_4$Alkyl und $R_2$ Wasserstoff, $C_1$-$C_4$Alkyl oder Phenyl sind.

8. Eine Verbindung der Formel I gemäss Anspruch 2, ausgewählt aus Soraphen A-5-on, Soraphen B-5-on und 9,10-Dihydro-Soraphen A-5-on.

9. Verfahren zur Herstellung von Verbindungen der Formel I gemäss Anspruch 1, dadurch

gekennzeichnet, dass man bei gegebenenfalls geschützten funktionellen Gruppen in einer macrocyclischen Verbindung der Formel

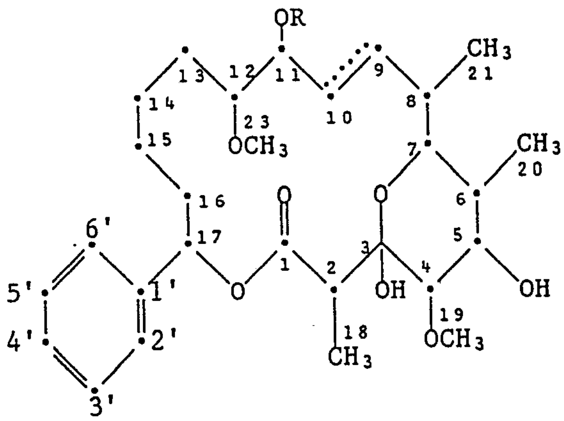

in der die gepunktete Linie in 9,10-Stellung wahlweise eine gesättigte Bindung oder eine Doppelbindung bedeutet mit der Massgabe, dass R Methyl bedeutet, wenn in 9,10-Stellung eine Doppelbindung vorliegt; die OH-Gruppe in 5-Stellung zur Ketogruppe oxidiert, und sofern gewünscht,

a) diese oximiert, und, sofern gewünscht, das Oximderivat durch Einführung des Substituenten Y verethert oder durch Einführung von -CO-Z acyliert, oder

b) die Ketogruppe mit einem Hydrazinderivat

$H_2N-N(R_1)(R_2)$

in ein Hydrazon, bzw. wenn $R_2$ Carbamoyl bedeutet, in ein Semicarbazon verwandelt;

unter gegebenenfalls weiterer Acylierung und/oder Abspaltung von Schutzgruppen; wobei die genannten Substituenten die für Formel I gegebene Bedeutung haben.

10. Mittel zur Bekämpfung oder Verhütung von Pflanzenkrankheiten, dadurch gekennzeichnet, dass es als mindestens einen Wirkstoff eine Verbindung der Formel I gemäss einem der Ansprüche 1 bis 8 enthält, zusammen mit einem geeigneten Trägermaterial.

11. Verwendung von Verbindungen der Formel I gemäss Anspruch 1 zur Bekämpfung bzw. Verhütung von Pflanzenkrankheiten.

12. Verfahren zur Bekämpfung oder Verhütung von Pflanzenkrankheiten, dadurch gekennzeichnet, dass mindestens eine Verbindung der Formel I gemäss Anspruch 1 auf die Pflanze, auf Pflanzenteile oder ihren Standort appliziert wird.